## Europäisches Patentamt

## European Patent Office

## Office européen des brevets

(19)

(11) Numéro de publication: **0 034 667**
A1

# (12) DEMANDE DE BREVET EUROPEEN

(21) Numéro de dépôt: **80200743.5**

(22) Date de dépôt: **04.08.80**

(51) Int. Cl.³: **C 12 P 5/02**
**C 02 F 11/04**

(30) Priorité: **26.02.80 BE 58427**

(43) Date de publication de la demande:
**02.09.81 Bulletin 81/35**

(84) Etats contractants désignés:
**AT CH DE FR GB IT LI LU NL SE**

(71) Demandeur: **STUDIEBUREAU O. DE KONINCKX**
**naamloze vennootschap**
**Valkenveld 1-12**
**B-2610 Wilrijk(BE)**

(71) Demandeur: **RIJKSUNIVERSITEIT GENT**
**Coupure 533**
**B-9000 Gent(BE)**

(72) Inventeur: **Verstraete, Willy**
**Dasstraat 4**
**B-9030 Wondelgem(BE)**

(74) Mandataire: **Donné, Eddy**
**M.F.J.Bockstael Arenbergstraat 13**
**B-2000 Anvers(BE)**

(54) Procédé de production de méthane à l'état gazeux.

(57) Procédé de production de méthane à l'état gazeux, caractérisé en ce que dans l'espace réactionnel, où s'écoule la réaction de fermentation productrice de méthane, règne constamment une certaine sous-pression.

EP 0 034 667 A1

Croydon Printing Company Ltd.

1

## "Procédé de production de méthane à l'état gazeux

La présente invention a trait à un procédé pour la production de méthane à l'état gazeux, c'est-à-dire à un procédé par lequel des matériaux organiques d'origine biologique ou xéno-biologique, qui se trouvent à l'état solide, liquide, dissous ou autre, sont transformés de manière connue en du méthane gazeux par des micro-organismes, à savoir, en particulier, par des bactéries ou d'autres micro-organismes unicellulaires, ou par des composés biochimiques qui en proviennent.

On sait que le méthane gazeux est un des produits de toutes sortes de processus ou réactions biochimiques, telles que

- la fermentation des boues séparées dans les stations d'épuration des eaux résiduaires;
- la fermentation des eaux résiduaires concentrées dans l'industrie biochimique et les industries apparentées;
- la fermentation de certaines fractions des déchets domestiques;
- la fermentation des déjections en provenance de l'élevage porcin, bovin ou autre;
- la fermentation de produits végétaux provenant de la culture de plantes spéciales, dites productrices d'énergie, telles que les plantes cultivées en vue de la production de méthane ou autres.

Dans ces nombreuses applications, la cuve à fermentation se présente sous de nombreuses formes d'exécution allant du réacteur-mélangeur conventionnel jusqu'au réacteur à contact anaérobie, au filtre anaérobie, au réacteur moderne à courant ascendant ou à tout autre dispositif moderne concerné.

On sait également que la fermentation productrice de méthane est normalement conduite de manière que le gaz est recueilli sous une faible surpression d'environ 10 à 100 cm de colonne d'eau, de sorte que dans la chambre à réaction règne une certaine pression de méthane gazeux.

Or, le procédé de production de méthane gazeux selon la présente invention prévoit, contrairement au procédé habituel universellement appliqué jusqu'à présent, que dans la chambre où s'écoule la réaction de fermentation productrice de méthane gazeux, règne constamment une sous-pression variant par exemple de 0 & 0,5 atmosphère, avec comme conséquence une accélération sensible, allant de 10 à 50%, de la réaction de fermentation. Cette sous-pression s'obtient par exemple par une captation ou une évacuation appropriée du méthane gazeux engendré.

Le procédé de production de méthane gazeux à sous-pression selon l'invention a été soigneusement élaboré, essayé et mis au point au laboratoire, et cela à des sous-pressions variant de un à cinq mètres de colonne d'eau, avec comme résultat une augmentation sensible de la production de méthane, due à la sous-pression, allant jusqu'à 50% (production exprimée en Nm3 de méthane par mètre cube de volume réactionnel et par jour).

Par conséquent, l'augmentation de la vitesse réactionnelle du à la sous-pression régnant dans le réacteur en cas d'application du procédé selon l'invention se traduit par une augmentation sensible de la production volumétrique de méthane gazeux par unité de temps (en $Nm^3/h$), ce qui signifie, en d'autres termes, une augmentation sensible du volume de gaz produit dans un temps déterminé, c'est-à-dire une amélioration appréciable du degré de transformation des matériaux organiques concernés et donc du rendement de l'installation et du procédé mis en oeuvre.

Dans la pratique industrielle on vise généralement la réalisation d'un degré de transformation déterminé, c'est-à-dire l'obtention d'un rendement favorable, et cela de manière que l'avantage de la conduite à sous-pression se traduit par une réduction sensible du volume réactionnel pour un même rendement. D'ailleurs, les résultats de calculs provisoires montrent que, pour un même rendement, le volume réactionnel pourra être réduit d'environ un tiers par rapport à celui des réacteurs conventionnels. Et comme les frais d'investissement pour la création d'une installation industrielle pour la production de méthane par fermentation se rapportent en majeure partie au réacteur, il va sans dire que toute réduction du volume réactionnel présente des avantages économiques considérables.

Revendications.

1.- Procédé de production de méthane à l'état gazeux, caractérisé en ce que dans l'espace réactionnel où s'écoule la réaction de fermentation productrice de méthane, règne constamment une certaine sous-pression.

2.- Procédé selon la revendication 1, caractérisé en ce que ladite sous-pression varie de 0 à 0,5 atmosphère.

3.- Procédé selon la revendication 1 ou 2, caractérisé en ce que ladite sous-pression s'obtient par aspiration du méthane gazeux engendré.

Office européen
des brevets

**RAPPORT DE RECHERCHE EUROPEENNE**

EP 80 20 0743

| Catégorie | DOCUMENTS CONSIDERES COMME PERTINENTS | | CLASSEMENT DE LA DEMANDE (Int. Cl.³) |
|---|---|---|---|
| | Citation du document avec indication, en cas de besoin, des parties pertinentes | Revendication concernée | |
| X | EP - A - 0 007 168 (GRAHAM-SNIDER ENERGY SYSTEMS)<br>* Document en entier * | 1-3 | C 12 P 5/02<br>C 02 F 11/04 |
| | -- | | |
| X | CHEMICAL ABSTRACTS, vol. 84, no. 25<br>21ème juin 1976, page 419, ref. 178201p<br>Columbus, Ohio, US<br>& JP - A - 75 135279 (HITACHI LTD) (27-10-1975)<br>* Résumé * | 1-3 | |
| | -- | | **DOMAINES TECHNIQUES RECHERCHES (Int. Cl.³)** |
| X | FR - A - 911 622 (A. VAUGEAN et al.)<br>* Page 5, ligne 89 - page 6, ligne 35; résumé 5a * | 1-3 | C 12 P 5/02<br>C 02 F 11/04<br>C 02 F 3/28 |
| | -- | | |
| X | GB - A - 521 036 (J.D. GRIFFIN)<br>* Document en entier * | 1-3 | |
| | ---- | | |

**CATEGORIE DES DOCUMENTS CITES**

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons

&: membre de la même famille, document correspondant

| | | | |
|---|---|---|---|
| | Le présent rapport de recherche a été établi pour toutes les revendications | | |
| Lieu de la recherche<br>La Haye | Date d'achèvement de la recherche<br>02-06-1981 | Examinateur<br>DESCAMPS | |

OEB Form 1503.1  06.78